# EUROPEAN PATENT SPECIFICATION

(11) **EP 2 552 464 B1**
(45) Date of publication and mention of the grant of the patent: **28.02.2018**
(21) Application number: 10718696.7
(22) Date of filing: 30.03.2010
(51) Int. Cl.: A61K 35/745, A61P 37/08

(54) **USE OF BIFIDOBACTERIA FOR PREVENTING ALLERGY IN BREASTFED INFANTS**
VERWENDUNG VON BIFIDOBAKTERIEN ZUR ALLERGIEPRÄVENTION IN GESTILLTEN SÄUGLINGEN
UTILISATION DE BIFIDOBACTERIES DESTINEE A LA PREVENTION D'ALLERGIES AUX NOURISSONS ALLAITES.

(43) Date of publication of application: 06.02.2013
(73) Proprietor: Assistance Publique - Hôpitaux de Paris, 75004 Paris (FR); Compagnie Gervais Danone, 75009 Paris (FR)
(72) Inventor: DUPONT, Christophe, F-92140 Clamart (FR); SOULAINES, Pascale, F-75012 Paris (FR); AUBERT-JACQUIN, Cécile, CH-1564 Domdidier (CH); LECROIX, Francis, F-59270 Godewaersvelde (FR)
(74) Representative: Gevers & Orès
(86) International application number: PCT/IB2010/001042
(87) International publication number: WO 2011/121379

(56) References cited:
- WO-A1-2009/074754
- WO-A1-2009/151330
- SANDY PENG ET AL: "Antiallergic effect of milk fermented with lactic acid bacteria in a murine animal model", JOURNAL OF AGRICULTURAL AND FOOD CHEMISTRY, vol. 55, no. 13, 27 May 2007 (2007-05-27), pages 5092-5096, XP009103004, AMERICAN CHEMICAL SOCIETY, US ISSN: 0021-8561, DOI: 10.1021/JF062869S
- DATABASE WPI Week 200939 Thomson Scientific, London, GB; AN 2009-G23489 XP002608154, -& CN 101 366 734 A (LIAONING DASHENG MEDICINE IND CO LTD) 18 February 2009 (2009-02-18)
- INOUE Y ET AL: "Suppressive effects of Bifidobacterium breve strain M-16V on T-helper type 2 immune responses in a murine model", BIOLOGICAL AND PHARMACEUTICAL BULLETIN, vol. 32, no. 4, 2009, pages 760-763, XP002608155, PHARMACEUTICAL SOCIETY OF JAPAN JPN DOI: 10.1248/BPB.32.760
- HEYMAN M ET AL: "Effects of specific lactic acid bacteria on the intestinal permeability to macromolecules and the inflammatory condition", ACTA PAEDIATRICA, vol. 94, no. Suppl. 449, 1 October 2005 (2005-10-01), pages 34-36, XP002480544, UNIVERSITETSFORLAGET, OSLO, NO ISSN: 0803-5253, DOI: 10.1080/08035320510043853
- TERPEND K ET AL: "Intestinal barrier function and cow's milk sensitization in guinea pigs fed milk or fermented milk", JOURNAL OF PEDIATRIC GASTROENTEROLOGY AND NUTRITION, vol. 28, no. 2, 1 February 1999 (1999-02-01), pages 191-198, XP009103003, RAVEN PRESS, NEW YORK, NY, US ISSN: 0277-2116, DOI: 10.1097/00005176-199902000-00019

## Description

### FIELD:

The present invention relates to the use of *Bifidobacteria* for the preparation of a fermented composition for use as a supplement for human milk for preventing allergy.

### BACKGROUND OF THE INVENTION:

For several decades, the incidence of allergy and other atopic diseases has increased in industrialized countries. Exposure to allergenic food during early infancy is an important risk factor for sensitization and later development of atopic diseases. One of the best ways to manage atopic disease, in particular allergy, remains prevention.

Infant formulae with extensively hydrolysed cow's milk protein or free amino acid, devoid of allergens, are used to treat allergy and other atopic diseases. To prevent allergy in infants at risk infant formulae are marketed which contain partially hydrolysed cow's milk protein.

Also breast feeding, which is the preferred way of feeding infants, is said to reduce the risk of allergy. However, breast milk is not always optimal to prevent allergy and other atopic diseases. Several studies have reported the absence of a protective effect of prolonged and exclusive breastfeeding on asthma or allergy. There is therefore a need for compositions for use in breast fed infants that improve the effects of breast milk on allergy and other atopic diseases. Hence, several compositions methods for preventing allergy have been proposed:
- International Application WO 01/97822 discloses a decrease in atopic eczema in breast fed infant ingesting *Lactobacillus* GG and whose mothers were given *Lactobacillus* GG 2 to 4 weeks before delivery;
- European Application EP2140771 relates to the use of a composition comprising a) at least two different microorganisms; or b) at least one microorganism and at least one indigestible oligosaccharide; or c) at least two different *Bifidobacteria* species, subspecies or strains for the manufacture of a composition for enteral administration to an infant delivered via caesarean section, in the aim of stimulating the development of a healthy intestinal flora. This composition can further be mixed with human breast milk;
- International Application WO 2009/074754 relates to the use of a *Bifidobacterium* strain for preparing compositions, particularly of an infant formula, for preventing and/or treating allergic manifestations. The bacteria, which preferably belong to the *Bifidobacterium breve* species, can be killed or withdrawn from the composition during said method;

- International Application WO 2008/153391 discloses an infant and/or toddler nutrition comprising non-viable *Bifidobacterium breve* and a non-digestible oligosaccharide and its use for prevention of allergy are disclosed;
- International Application WO 2009/151330 relates to methods and compositions for feeding infants delivered via caesarean section, in particular to the use of a product obtained by fermentation of milk, whey, whey protein, whey protein hydrolysate, casein, casein hydrolysate and/or lactose by lactic acid producing bacteria. Such a product can be used to decrease the incidence and severity of atopic diseases, such as allergy; and
- International Application WO 2009/151331 discloses a nutritional composition comprising a combination of non-digestible oligosaccharides and a product obtained by incubating an aqueous substrate by bifidobacteria and optionally a product obtained by incubating an aqueous substrate by *Streptococcus thermophilus.* Said combination synergistically improves the immune system by stimulating the Th1 response, and/or decreasing the Th2 response, improving the vaccination response, improving the resistance against infections and/or decreasing allergy.

Further, the use of *Bifidobacterium breve* in combination with human breast milk to improve intestinal flora is known:
- Satoh et al. (2007, Int J Prob Preb 2:149-154) discloses the use of a *B. breve* strain supplemented to human breast milk for preterm infants to prevent infections and particularly necrotizing enterocolitis; and
- Campeotto et al. (2008, 9th Biennial Congress of Anaerobe Society of the Americas, Califiornia USA, June 24-27) disclose a method of enhancing mucosal immunity in a human preterm infant, receiving a preterm formula and optionally breast milk, comprising the step of administering heat killed *B. breve* C50 and *S*. *thermophilus* to said infant.

### SUMMARY OF THE INVENTION

The inventors have found that when a composition, comprising a composition fermented and/or bioconversed with a strain of *Bifidobacterium breve,* was administered to infants less allergic manifestations were observed in these infants compared to infants not receiving this composition. Unexpectedly, this observed beneficial effect was present to a much higher degree in infants who also received breast feeding than in non-breast fed infants, which exclusively received the composition. Furthermore, the onset of allergic manifestations was delayed in infants receiving a composition fermented with *Bifidobacterium breve* in supplement to breastmilk, compared to infants exclusively fed breastmilk. This beneficial effect was observed regarding allergic manifestations related to the respiratory tract, but most pronouncedly regarding allergic manifestations related to the gastro-intestinal tract.

These results show that a synergy occurs between a composition fermented and/or bioconversed with a strain of *Bifidobacterium breve* and breast milk. The present invention therefore is advantageously used as a supplement for breast fed infants.

### DETAILED DESCRIPTION OF THE INVENTION

The present invention is defined in appended claims 1-5 The invention pertains to a composition comprising a strain of *Bifidobacterium breve* and a strain of *Streptococcus thermophilus,* wherein the composition is obtained by a process comprising a step of fermentation and/or bioconversion of a milk substrate in the presence of said *Bifidobacterium breve* strain for use in delaying the onset of allergy at least later than 15 days after birth, in a human infant receiving human milk at least once a day, wherein said composition is administered daily from birth until the infant is at least 15 days of age, characterized in that the strain of *B. breve* is strain *B. breve* CNCM I-2219 and the strain of *S. thermophilus* is strain *S. thermophilus* CNCM 1-1620.

The inventors disclose a composition comprising a strain of *Bifidobacterium breve,* wherein the composition is obtained by a process comprising a step of fermentation and/or bioconversion of a milk substrate in the presence of said *Bifidobacterium breve* strain for use in preventing and/or treating and/or delaying the onset of allergy, preferably in delaying the onset of allergy, in a human infant receiving human milk.

The inventors also disclose an unit dose or container comprising a dietary supplement comprising a strain of *Bifidobacterium breve,* wherein the dietary supplement is obtained by a process comprising a step of fermentation and/or bioconversion of a milk substrate in the presence of said *Bifidobacterium breve* strain, and wherein the composition is either a powder in an amount of from about 0.5 gram to about 20 grams per unit dose or a sterilized liquid with a volume of about 0.5 to 20 ml.

The inventors further disclose a method of preventing and/or treating and/or delaying the onset allergy in a human infant, said method comprising the steps of
a. administering on one day human breast milk to said human infant, and
b. administering on the same day a dietary supplement comprising a strain of *Bifidobacterium breve,* wherein the dietary supplement is obtained by a process comprising a step of fermentation and/or bioconversion of a milk substrate in the presence of said *Bifidobacterium breve* strain.

### Composition

The inventors disclose a composition comprising a milk substrate fermented and/or bioconversed with a strain of *Bifidobacterium breve.*

*Bifidobacterium breve* is a Gram-positive, anaerobic, branched rod-shaped bacterium.

Preferably, in the herein disclosed composition, the 16S rRNA sequence of said *B. breve* has at least 95 % identity with the full-length sequence of the 16S rRNA of the type strain of *B. breve* ATCC 15700, more preferably at least 97 % identity (Stackebrandt & Goebel, 1994, Int. J. Syst. Bacteriol. 44:846-849). The *Bifidobacterium* included in the composition and methods and uses herein disclosed preferably hybridises with the *B. breve* probe and gives a signal with the 5' nuclease assay method as described in WO 2005/039319. Preferred *B. breve* strains are those isolated from the faeces of healthy human milk-fed infants. Typically, these are commercially available from producers of lactic acid bacteria, but they can also directly be isolated from faeces, identified, characterised and produced.

Yet preferably, the herein disclosed composition contains at least one *B. breve* selected from the group consisting of *B. breve* Bb-03 (Danisco), *B. breve* M-16V (Morinaga), *B. breve* R0070 (Institute Rosell, Lallemand), *B. breve* BR03 (Probiotical), *B. breve* BR92 (Cell Biotech), *B. breve* DSM 20091, *B. breve* LMG 11613 and *B. breve* CNCM 1-2219. Preferably, the *B. breve* is *B. breve* M-16V (Morinaga). Yet more preferably, in the herein disclosed composition, the strain of *B. breve* is *B. breve* CNCM 1-2219, deposited in accordance with the Budapest Treaty, on May 31, 1999, at the Collection Nationale de Cultures de Microorganismes held by Institut Pasteur, 25 rue du Docteur Roux, in Paris, and described in International Application WO 01/01785.

Advantageously, a process for preparing the composition according to the present invention, as well as the composition herein disclosed, comprises a step of fermentation and/or bioconversion of a milk substrate in the presence of a strain of *Bifidobacterium breve* as defined above.

In a preferred embodiment, the composition according to the invention, as well as the composition herein disclosed, comprises a fermented milk substrate.

In another embodiment, the composition according to the invention, as well as the composition herein disclosed, comprises a milk-derived product fermented by *Bifidobacterium breve,* of which the cells are inactivated after fermentation. The milk derived product which has been fermented and/or bioconversed by *B. breve* comprises fragments of or/and products excreted by *B. breve,* such as glycoproteins, glycolipids, peptidoglycan, lipoteichoic acid (LTA), lipoproteins, capsular polysaccharides and/or DNA. These molecules have an additional advantageous effect on the prevention or delay of onset of allergic manifestations. Furthermore, upon fermentation and/or bioconversion of *B. breve* with the milk-derived products, additional bio-active compounds may be formed, such as bioactive peptides and/or oligosaccharides, which also may have an advantageous effect on the prevention or delay of onset of allergic manifestations.

This step of fermentation and/or bioconversion of a milk substrate can be performed under aerobic or anaerobic conditions.

As used herein the term "milk substrate" refers to a liquid medium comprising at least a whey protein fraction and lactose. Whey proteins can be hydrolysed and/or lactalbumin enriched. Whey filtrate can also be added to the milk substrate. Non limitative examples of milk substrates include milk, milk concentrate, a base for infant milk food, a base for yogurt, and the like.

In another preferred embodiment, the composition according to the invention, as well as the composition herein disclosed, is obtained by a process comprising a step of bioconversion of a milk substrate by the strain of *Bifidobacterium breve,* performed under conditions which are unfavorable to production of acid by said strain, as described in International Applications WO 01/01785 and WO 2009/074754. This bioconversion which corresponds to a non-acid or reduced-acid fermentation also allows production of active metabolites having a beneficial health effect, in particular on allergy. Particular conditions of this fermentation and/or bioconversion are described below.

As used herein the term "conditions which are unfavorable to production of acid by the strain of *Bifidobacterium breve"* refers to conditions under which the acidification of the medium by *Bifidobacterium breve* does not exceed 0.5 pH units during 8 hours of incubation for an initial inoculation of 1.10⁷ cfu per ml. These conditions can be easily determined by persons skilled in the art with the aid of routine trials, by varying in particular the aeration of the culture medium, its osmotic pressure and/or the culture temperature, and by measuring the pH at the beginning and at the end of the culture.

For a large number of *Bifidobacterium breve,* such conditions may be obtained in particular by:
- using a bacterial population from an inoculum in a phase posterior to the exponential growth phase;
- incubating under aerobic conditions, for example with stirring;
- incubating in the medium at an osmotic pressure corresponding to a water activity (AW) of 0.93 to 0.97; or
- incubating at a temperature of 40 to 48 °C.;
as well as combinations of these various conditions.

In this embodiment, the milk substance per ml of milk substrate, and the final *Bifidobacterium* population at the end of the bioconversion reaction preferably may be comprised between 1.10⁵ to 1.10⁹ cfu per ml of product.

The pH of the milk substrate during the bioconversion and/or fermentation step with the *Bifidobacteria* is preferably 6.0 to 7 and the pH of the product at the end of the bioconversion and/or fermentation is preferably from 5.8 to 7.

Depending on the conditions used, the time duration of fermentation and/or bioconversion, *i.e.* the time of contact between the milk substrate and the bacteria, is preferably from 4 to 24 hours.

Of course, in a process according to the invention, the steps of bioconversion and/or fermentation as described above are not exclusive. By way of example, such a process can comprise a step of bioconversion (under non-acid or reduced-acid conditions) of a milk substrate by *Bifidobacterium breve,* followed by a step of acid fermentation by this bacterium. Alternatively, both reactions (bioconversion and fermentation) can be carried out in parallel, on two distinct milk substrates; then, the products obtained from these two reactions being brought together in the final composition.

According to another advantageous embodiment, the product(s) obtained from the bioconversion and/or fermentation process as described above can be subjected to various treatments. Particularly, a step of inactivation of the bacteria can be applied to these products to obtain shelf-stable products. Preferably, the product(s) obtained from the bioconversion and/or fermentation process is(are) heated to inactivate the bacteria.

The composition according to the invention, as well as the composition herein disclosed, comprises preferably an amount of non-viable *Bifidobacterium breve* equivalent to at least 10³ cfu per gram dry weight of the composition. The composition according to the invention, as well as the composition herein disclosed, preferably comprises non-viable *B. breve* in an equivalent of 10³ to 10¹³ cfu *B. breve* per gram dry weight of the present composition, preferably 10⁴ to 10¹², more preferably 10⁵ to 10¹¹, most preferably the equivalent of 10⁵ to 10¹⁰ cfu *B. breve* per gram dry weight of the present composition. Preferably, the composition according to the invention, as well as the composition herein disclosed, comprises non-viable *B. breve* in an equivalent of 10⁴ to 10¹³, more preferably from 10⁵ to 10¹², most preferably the equivalent of 10⁶ to 5x10¹¹ cfu *B. breve* per gram of the total of non-digestible oligosaccharide. The dose of non-viable *B. breve* according to the present invention is preferably administered at a daily dose of the equivalent of 10⁴ to 10¹⁴, more preferably from 10⁵ to 10¹³, even more preferably the equivalent of 10⁶ to 10¹² , most preferably 10⁸ to 5x10¹¹ cfu. When the composition is a liquid, the composition preferably comprises the equivalent of 10⁴ to 10¹⁴ cfu of non-viable *B. breve* per 100 ml of the present composition, preferably 10⁵ to 10¹³, more preferably 10⁶ to 10¹², most preferably the equivalent of 10⁶ to 10¹¹ cfu *B. breve* per 100 ml of the present composition.

An amount of non-viable *Bifidobacterium breve* equivalent to at least 10³ cfu per gram dry weight means non-viable *Bifidobacterium breve* in an amount which is the equivalence of an amount of at least 10³ cfu *B. breve* per gram dry weight. The equivalent of cfu can suitably be determined by performing the 5' nuclease assay with the *B. breve* probes and primers as disclosed in International Application WO 2005/039319 in the product (*i.e.* an infant formula) comprising non-viable *B. breve* and comparing this with a calibration curve obtained from a comparable product (for instance a standard infant formula) to which known amounts of dried, viable *B. breve* cfu have been added. The dried viable bifidobacteria can be commercially obtained as described above. The value of cfu in the calibration curve made by viable or living *B. breve* which has the same 5' nuclease assay response as the product comprising the inactivated *B. breve* is considered to be the equivalent amount in cfu of non-viable *B. breve.* Alternatively, the amount of cfu per gram dry weight can be determined in a composition just before the inactivation step.

The presence of a high amount of viable bifidobacteria would still result in post-acidification in liquid infant formulae. The composition according to the invention, as well as the composition herein disclosed, therefore comprises less than 10³ cfu viable bifidobacteria, preferably less than 10², more preferably less than 10 cfu per gram dry weight of the composition. When in the form of a liquid, the composition according to the invention, as well as the composition herein disclosed, comprises preferably less than 10⁴ cfu viable bifidobacteria, preferably less than 10³, more preferably less than 10² cfu per 100 ml. The cfu of viable bifidobacteria can suitably be determined as described in Ingham, S. C., 1999 (J. Food Prot. 62 (1) p.77-80). Preferably the composition according to the invention, as well as the composition herein disclosed, comprises no measurable living *B. breve* cfu at all. Total absence of living *B. breve* is however difficult or cannot be determined. Suitably, the absence of living *B. breve* by plating methods can be determined as being below the detection limit. This detection limit is 10³ cfu/g dry weight of composition.

Non-viable *B. breve* according to the present invention relates to dead *B. breve,* non-culturable *B. breve,* non-growing *B. breve* and/or (metabolically) inactive *B. breve. B. breve* cells can be made non-viable by methods known in the art, including heat treatment steps (including sterilization, pasteurization, UHT treatment), radiation (UV), treatment with oxygen, treatment with bactericidals such as ethanol, sonication, ultra high pressure application, high pressure homogenization and use of a cell disruptor. Preferably the *B. breve* is heat-killed.

The fermented and/or bioconversed product of the present invention preferably with non-viable *B. breve* still has the advantageous effect on allergic manifestations, while providing many product technological benefits, including increased shelf-life, improved safety by a reduced incidence of bacterial contamination, decreased post-acidification of the product, improved dosage control and improved convenience of reconstitution.

In the herein disclosed composition, a strain of *Streptococcus thermophilus* is preferably also used for the preparation of said composition. *Bifidobacterium breve* is a Gram-positive, anaerobic, branched rod-shaped bacterium.

Yet preferably, in the herein disclosed composition, the 16S rRNA sequence of said *S. thermophilus* has at least 95 % identity with the full-length sequence of the 16S rRNA of the type strain of *S thermophilus* ATCC 19258, more preferably at least 97 % identity.

Yet more preferably, in the herein disclosed composition, the strain of *S*. *thermophilus* is *S. thermophilus* I-1620, deposited in accordance with the Budapest Treaty, on August 23, 1995, under the number I-1620 at the CNCM (Collection Nationale de Cultures de Microorganismes) held by Institut Pasteur, 25 rue du Docteur Roux, in Paris. The presence of at least two strains advantageously synergistically improves the effect on prevention or delay of onset of allergic manifestations.

According to another advantageous embodiment of the present invention, composition according to the invention, as well as the composition herein disclosed,, obtainable by the process as described above, can also comprise a fraction obtained by fermentation and/or bioconversion of a milk substrate or lactose by a *Streptococcus thermophilus* strain as defined above. This fraction can be advantageously obtained by the process described in the International Application WO 96/06924.

Preferably the amount of *S. thermophilus* is the same as for *B. breve.* For reasons similar to *B. breve* described above, the cells are preferably used to ferment or to bioconverse a milk substrate as defined above or lactose. For reasons similar to *B. breve* described above, the cells are preferably present in activated form. The ratio between the final *B. breve* population and the final *S. thermophilus* population based on cfu at the end of the fermentation and/or bioconversion reaction is preferably between 10⁵ to 10¹, more preferably between 10⁴ to 10².

The composition according to the present invention may be consumed as it is, or may be subjected to various treatments whose nature varies according to the ready-to-eat product which it is desired to obtain. It may, for example, be supplemented with texturing agents, flavoring agents, vitamin or mineral supplements, fat and the like, if these were not added to the original medium. It may also be concentrated or diluted.

Preferably, the dietary supplement is a product obtained after fermentation and/or bioconversion of a milk substrate by a strain of *B. breve,* optionally with *S*. *thermophilus* and optionally with an inactivation step as described in more detail in the section above, without any further addition. Such a dietary supplement would interfere to a lesser degree with the breast feeding than when further additions were made, since it contains the bioactive components in the highest concentration.

The present dietary supplement is preferably a complete infant formula. Such food preferably comprises lipid, protein and carbohydrate and is preferably administered in liquid form. Preferably the dietary supplement is a ready-to-use liquid food, *e.g.* is in a ready-to-feed liquid form. A packed ready-to-use liquid food advantageously involves less steps for preparation than a powder to be reconstituted and hence a reduced chance on contamination by harmful micro-organisms. In case of use for preterm infants the infant formula preferably is a pretem infant formula.

Hence, a dietary supplement of the present invention preferably comprises between 5 and 50 % lipid, between 5 and 50 % protein, between 15 and 90 % carbohydrate based on total calories. More preferably the present infant nutrition comprises between 35 and 50 % lipid, between 7.5 and 12.5 % protein and between 35 and 80 % carbohydrate based on total calories. Preferably the present dietary supplement comprises lipids, more preferably vegetable lipids.

Preferably the present dietary supplement comprises proteins. The proteins used in the preparation are preferably selected from the group consisting of non-human animal proteins (such as milk proteins, including caseins and whey proteins, meat proteins and egg proteins), vegetable proteins (such as soy protein, wheat protein, rice protein, potato protein and pea protein), hydrolysates (partially and/or extensively), free amino acids and mixtures thereof. Cow's milk derived nitrogen source, particularly cow's milk protein proteins such as casein and whey proteins are particularly preferred, as the amino acid composition of these proteins is well balanced. Whey proteins may be derived from sweet whey or acid whey. The term cow's milk protein refers to proteins naturally found in cow's milk, *i.e.* casein, alpha-lactalbumin, lactoferirin, serum albumin, beta lactoglobulin. As the present composition is suitably used to reduce the allergic reaction in an infant and/or toddler, the protein of the infant nutrition is preferably selected from the group consisting of hydrolysed milk protein (e.g. hydrolysed casein and/or hydrolysed whey protein), hydrolysed vegetable protein and/or amino acids. The use of these proteins further reduces the allergic reactions of the infant and/or increases protein absorption. Preferably the protein source is extensively and/or partially hydrolysed. More preferably the protein source is extensively hydrolysed whey protein derived from cow's milk.

Preferably the present dietary supplement comprises digestible carbohydrates. The digestible carbohydrates used in the nutritional preparation are preferably selected from the group consisting of sucrose, lactose, maltose, galactose, glucose, fructose, corn syrup solids, starch and maltodextrins, and mixtures thereof, more preferably lactose.

Stool irregularities (e.g. hard stools, insufficient stool volume, diarrhoea) is a major problem in many babies. It was found that stool problems may be reduced by administering the present dietary supplement which has, in liquid ready to use form, an osmolality between 50 and 500 mOsm/kg, more preferably between 100 and 400 mOsm/kg.

In view of the above, it is also important that the dietary supplement does not have an excessive caloric density, however still provides sufficient calories to feed the subject. Hence, the dietary supplement, when in liquid ready to use form preferably has a caloric density between 0.1 and 2.5 kcal/ml, even more preferably a caloric density of between 0.4 and 1.2 kcal/ml, most preferably between 0.55 and 0.75 kcal/ml.

The (fresh, sterilized or reconstituted) dietary supplement according to the invention preferably has a pH of 6 to 7.5, more preferably of 6.5 to 6.9.

The present dietary supplement preferably has a viscosity between 1 and 60 mPa.s, preferably between 1 and 20 mPa.s, more preferably between 1 and 10 mPa.s, most preferably between 1 and 6 mPa.s, when in liquid, ready to use form. The low viscosity ensures a proper administration of the liquid, e.g. a proper passage through the hole of a nipple. Also this viscosity closely resembles the viscosity of human milk. Furthermore, a low viscosity results in a normal gastric emptying and a better energy intake, which is essential for infants and/or toddlers which need the energy for optimal growth and development. The present composition is preferably prepared by admixing a powdered composition comprising with water. Normally infant formula is prepared in such way. The present invention thus also relates to a packaged power composition wherein said package is provided with instruction to admix the powder with a suitable amount of liquid, thereby resulting in a liquid composition with a viscosity between 1 and 60 mPa.s. The viscosity of the liquid is determined using a Physica Rheometer MCR 300 (Physica Messtechnik GmbH, Ostfilden, Germany) at shear rate of 95 s⁻¹ at 20 °C.

The present invention also covers fresh, sterilized or dehydrated compositions as defined above. In a preferred embodiment of the invention, said composition is a pharmaceutical composition or a human milk fortifier (HMF).

Preferably the dietary supplement is a human milk fortifier. Preferably the composition is packed in a unit dose in the form of a powder in an amount of from about 0.5 to about 20 grams per unit dose, more preferably from 1 to 5 grams per unit dose. Preferably the composition is packed in the form of a sterilized liquid with a volume of about 0.5 to 10 ml, more preferably 1 to 5 ml. Preferably the unit dose is packaged with instructions for administering the composition in addition to human milk.

### Infant

Infants as defined in the present invention relate to human subjects with an age of 0 to 36 months, preferably 0 to 12 months, most preferably between 0 to 6 months.

Breast fed infants as defined in the present invention relate to infants receiving at least once a day a portion of human milk. This human milk may be administered directly from the breast of the mother, but may also be administered via a bottle after expression of human breast milk.

In a particular embodiment of the invention, the infant is a premature infant. A premature (or preterm) infant relates to an infant born before the standard period of pregnancy is completed before or on 37 weeks pregnancy of the mother, *i.e*. before or on 37 weeks from the beginning of the last menstrual period of the mother. Typically the birth weight of such infants is low and less than 2500 grams at birth. Preterm infants are very vulnerable, especially in the first week after birth. Therefore the prevention or delay of onset of allergic manifestations is particular advantage for this group of infants.

It was found by the inventors that the composition of the present invention advantageously delays the onset of allergy in breast fed infants. Therefore, in a preferred embodiment of the invention, the infant is an infant at risk of developing an allergy. An infant at risk of allergy or atopic disease in the present invention relates to an infant having more than one family member and at least one parent suffering from an atopic disease selected from asthma, allergic rhinitis, atopic dermatitis and food allergy.

### Application

The present infant composition is preferably enterally administered, more preferably orally.

The infant composition (or dietary supplement) is administered at least once a day to the infant in need thereof. However, the frequency of administration and the dosage of said composition depend, for example, upon the delivery vehicle and the infant to whom the composition is administered. It is within the level of knowledge of one of skill in the art to determine the frequency of administration and the dosage of said composition according the infant to whom it is administered.

The composition is administered daily from birth until the infant is at least 15 days of age, more preferably until the infant is at least 2 months of age.

Delay of onset of allergy relates to delay of onset of a disorder selected form the group consisting of food allergy, allergy to aeroallergens, atopic disease, manifestations of allergy and manifestations of atopic disease. Manifestations of allergy and/or atopic disease relate to allergic or atopic manifestations or of the skin, of digestive tract and /or of respiratory tract, more preferably of the digestive tract and/or the respiratory tract, most preferably of the digestive tract. Manifestations of the skin are preferably selected form the group consisting of atopic dermatitis, urticaria, angioedema, eczema, dry skin when associated with another symptom, rash, and pruritis. Manifestations of the digestive tract are preferably selected from the vomiting, gastro-esophageal reflux, acute diarrhea, colitis, abdominal pain, abdominal distention, rectal bleeding, fainting, transient hypotonia and failure to thrive. Respiratory tract symptoms are preferably selected from the group consisting of asthma, wheezing, wheezing bronchitis and spastic bronchitis.

Delay of onset in the present invention relates to the appearance of allergy at least later than 15 days, more preferably later than 60 days later, even more preferably later than 90 days after birth or compared to a control group not receiving the composition of the present invention.

Food allergy preferably relates to a food allergy selected form the group consisting of cow's milk protein allergy, egg white allergy, cod allergy, wheat flour allergy, soy bean flour allergy and peanut allergy. Allergy against aereoalergens is preferably selected form the group consisting of cat allergy, dust mite allergy, dog allergy, birch pollen allergy, grass pollen allergy, and *Alternaria* allergy. Food allergy and allergy against aeroallergens can be determined as known in the art, for example by prock0in-prock tests, atopy patch tests, or determination of specific IgE levels (>5 kU/l).

Preferably, the supplement represents less than 25, more preferably less than 10, even more preferably less than 5 wt.% of the total protein, based on daily intake. The other protein is preferably derived from the human milk.

### EXAMPLE 1: B. BREVE CNCM 1-2219 AND S. THERMOPHILUS CNCM 1-1620 HEAT KILLED AFTER A FERMENTATION PROCESS, REDUCE THE MUCOSAL INITIAL MANIFESTATIONS OF FOOD ALLERGY

A study has been performed to assess the effect of a composition, according to the invention, comprising *B. breve* CNCM 1-2219 and *S. thermophilus* CNCM I-1620, heat killed after a fermentation process, on allergy in infants (breast fed or not).

### 1.1- Materials and Methods

In this study, infants were either exclusively
1 breastfed,
2 fed with a standard infant formula which does not comprise B. breve or a fermented milk substrate, namely Gallia® (sold by Gallia, France),
3 fed with the infant formula Calisma® (sold by Gallia, France) which comprises the strains *B. breve* CNCM I-2219 and *S. thermophilus* CNCM 1-1620, heat killed after a fermentation process,
4 breastfed and fed with the infant formula Gallia® , or
5 breastfed and fed with Calisma®.

The average quantity of formula given to partially breast fed infants was 600 ml at the end of the intervention period. The infants were at risk for allergy, with at least two family members suffering from atopy, of which at least one parent. Atopy in this respect relates to asthma, allergic rhinitis, atopic dermatitis and/or food allergy. The mothers consumed at least 500 ml per day of either Gallia (group 4) or Calisma (group 5), during lactation, and did not consume any other milk, fermented product or priobiotic product. The results were analysed in terms of clinical symptoms that can be attributed to allergy, *i.e.* allergic manifestations or potential allergic manifestations. They include the gut and respiratory tract in infants at the age of 12 months.

These symptoms were divided into three categories: i) cutaneous symptoms (atopic dermatitis, urticaria, angioedema, eczema, dry skin when associated with another symptom, rash, and pruritis, ii) digestive symptoms including upper digestive symptoms (vomiting and gastro-esophageal reflux), and lower digestive symptoms (acute diarrhea, colitis, abdominal pain, abdominal distention, rectal bleeding) and general symptoms associated (fainting or transient hypotonia and failure to thrive), and iii) respiratory symptoms (asthma, wheezing, wheezing bronchitis and spastic bronchitis).

### 1.2- Results

Results are shown in Table 1 below. Infants fed with Calisma® and breastfed infants receiving also Calisma® exhibited less allergic manifestations at 4 months follow up. These effects were observed regarding symptoms of the gastro-intestinal tract and regarding the respiratory tract, most pronouncedly gastro-intestinal tract (see Table 2 below). The infants exclusively fed with Infant Milk Formula (IMF), namely Calisma or Gallia, and breast fed infants fed with Gallia tended to be statistically different from the breast fed infant group.

However, the breast fed infants fed with Calisma were not statistically different from the breast fed group regarding total allergic manifestations. However, surprisingly, in this group a delayed onset of potential allergic manifestations was observed compared to exclusively breast fed infants during the first 3 months, more pronouncedly during the first 75 days. Gastro-intestinal tract related allergic manifestations were reduced in breast fed infants also fed Calisma compared with exclusively control or Calisma fed infants and also compared with breast fed infants also fed standard IMF and were even lower than in completely breast fed infants (Table 2).

**Table 1: Cumulative incidence of potential allergic manifestations in time in infants fed standard IMF, an IMF of the present invention in partially breast fed infants and in exclusively IMF fed infants. Incidence is percentage of infants having a potential allergic manifestation relative to total infant of the feeding group.**

| Period up to day: | Infant feeding groups | | | | |
|---|---|---|---|---|---|
| | Exclusively IMF fed | | (Partially) Breast fed infants | | |
| | Standard IMF | Calisma | Standard IMF | Calisma | - |
| 30 | 36 | 21 | 0 | 0 | 9 |
| 45 | 43 | 38 | 33 | 0 | 15 |
| 60 | 61 | 62 | 56 | 7 | 21 |
| 75 | 61 | 65 | 67 | 7 | 29 |
| 90 | 71 | 74 | 67 | 29 | 35 |
| 105 | 82 | 76 | 94 | 57 | 44 |
| 120 | 93 | 82 | 100 | 57 | 53 |

**Table 2: Cumulative incidence of gastro-intestinal potential allergic manifestations at day 120 in infants fed standard IMF, an IMF of the present invention in partially breast fed infants and in exclusively IMF fed infants. Incidence is percentage of infants having a potential gastro-intestinal allergic manifestation relative to total infant of the feeding group.**

| Allergic manifestation (yes/no) | | Infant feeding group | | | | |
|---|---|---|---|---|---|---|
| | | Exclusively IMF fed | | (Partially) Breast fed | | |
| | | Standard IMF | Calisma | Standard IMF | Calisma | - |
| No | N | 14 | 16 | 9 | 11 | 25 |
| | % | 50.00 | 47.06 | 50.00 | 78.57 | 73.53 |
| Yes | N | 14 | 18 | 9 | 3 | 9 |
| | % | 50.00 | 52.94 | 50.00 | 21.43 | 26.47 |

## Claims

1. Composition comprising a strain of *Bifidobacterium breve* and a strain of *Streptococcus thermophilus,* wherein the composition is obtained by a process comprising a step of fermentation and/or bioconversion of a milk substrate in the presence of said *Bifidobacterium breve* strain for use in delaying the onset of allergy at least later than 15 days after birth, in a human infant receiving human milk at least once a day, wherein said composition is administered daily from birth until the infant is at least 15 days of age, **characterized in that** the strain of *B. breve* is strain *B. breve* CNCM 1-2219 and the strain of *S*. *thermophilus* is strain *S. thermophilus* CNCM I-1620.

2. Composition for use according to claim 1, **characterized in that** said allergy is selected from the group consisting of manifestations of allergies or atopic diseases in the digestive tract and the respiratory system.

3. Composition for use according to claim 1 or claim 2, **characterized in that** the infant is a premature infant and/or an infant at risk of allergy.

4. Composition for use according to any of claims 1 to 3, **characterized in that** said process comprises a step of inactivation of said strain at the end of the fermentation and/or bioconversion.

5. Composition for use according to any of claims 1 to 4, **characterized in that** said composition is for use in delaying the onset of allergy at least later than 60 days after birth and **in that** it is administered daily for a period of at least 2 months.

## Patentansprüche

1. Zusammensetzung, umfassend einen Stamm von *Bifidobacterium breve* und einen Stamm von *Streptococcus thermophilus,* wobei die Zusammensetzung durch einen Prozess erhalten wird, der einen Schritt des Fermentierens und/oder Biokonvertierens eines Milchsubstrats in Gegenwart des *Bifidobacterium* breve-Stammes zur Verwendung beim Verzögern des Beginns von Allergie auf mindestens später als 15 Tage nach der Geburt bei einem menschlichen Säugling, der mindestens einmal am Tag Frauenmilch empfängt, umfasst, wobei die Zusammensetzung ab der Geburt bis zum Alter von mindestens 15 Tagen des Säuglings täglich verabreicht wird, **dadurch gekennzeichnet, dass** der Stamm von *B. breve* der *B.* breve-Stamm CNCM I-2219 ist, und der Stamm von *S. thermophilus* der *S*. *thermophilus-Stamm* CNCM I-1620 ist.

2. Zusammensetzung zur Verwendung nach Anspruch 1, **dadurch gekennzeichnet, dass** die Allergie aus der Gruppe bestehend aus Manifestationen von Allergien oder atopischen Krankheiten im Verdauungstrakt und den Atemwegen ausgewählt ist.

3. Zusammensetzung zur Verwendung nach Anspruch 1 oder 2, **dadurch gekennzeichnet, dass** der Säugling eine Frühgeburt und/oder ein Säugling mit Allergierisiko ist.

4. Zusammensetzung zur Verwendung nach einem der Ansprüche 1 bis 3, **dadurch gekennzeichnet, dass** der Prozess einen Schritt des Inaktivierens des Stammes am Ende des Fermentierens und/oder Biokonvertierens umfasst.

5. Zusammensetzung zur Verwendung nach einem der Ansprüche 1 bis 4, **dadurch gekennzeichnet, dass** die Zusammensetzung zur Verwendung beim Verzögern des Beginns von Allergie auf mindestens später als 60 Tage nach der Geburt ist, und dadurch, dass sie für einen Zeitraum von mindestens 2 Monaten täglich verabreicht wird.

## Revendications

1. Composition comprenant une souche de *Bifidobacterium breve* et une souche de *Streptococcus thermophilus,* dans laquelle la composition est obtenue par un procédé comprenant une étape de fermentation et/ou de bioconversion d'un substrat laitier en présence de ladite souche de *Bifidobacterium breve* pour son utilisation pour retarder d'au moins plus de 15 jours après la naissance le déclenchement d'une allergie chez un nourrisson humain recevant du lait humain au moins une fois par jour, dans laquelle ladite composition est administrée quotidiennement depuis la naissance jusqu'à ce que le nourrisson ait au moins 15 jours, **caractérisée en ce que** la souche de *B. breve* est une souche de *B. breve* CNCM 1-2219 et la souche de *S. thermophilus* est la souche *S. thermophilus* CNCM I-1620.

2. Composition pour son utilisation selon la revendication 1, **caractérisée en ce que** ladite allergie est choisie dans le groupe consistant en manifestations d'allergies ou maladies atopiques des voies digestives et du système respiratoire.

3. Composition pour son utilisation selon la revendication 1 ou la revendication 2, **caractérisée en ce que** le nourrisson est un prématuré et/ou un nourrisson risquant de présenter des allergies.

4. Composition pour son utilisation selon l'une quelconque des revendications 1 à 3, **caractérisée en ce que** ledit procédé comprend une étape d'inactivation de ladite souche à la fin de la fermentation et/ou de la bioconversion.

5. Composition pour son utilisation selon l'une quelconque des revendications 1 à 4, **caractérisée en ce que** ladite composition est destinée à être utilisée pour retarder d'au moins plus de 60 jours après la naissance le déclenchement d'une allergie, et qui est administrée quotidiennement pendant une période d'au moins 2 mois.
